# EUROPEAN PATENT SPECIFICATION

(11) **EP 0 546 272 B1**
(45) Date of publication and mention of the grant of the patent: **28.02.1996**
(21) Application number: 92117267.2
(22) Date of filing: 09.10.1992
(51) Int. Cl.: C07C 323/58, A61K 31/195, A61K 31/205

(54) **S-carboxymethylcysteine lysine salt monohydrate and a process for the preparation thereof**
5-Carboxymethylcysteine Lysinsalz-Monohydrat und Verfahren zu seiner Herstellung
Sel de 5-carboxy-méthyl cystéine lysine monohydraté et procédé pour sa préparation

(30) Priority: 12.12.1991 IT MI913338
(43) Date of publication of application: 16.06.1993
(73) Proprietor: DOMPE' FARMACEUTICI S.p.A., I-20122 Milano (IT)
(72) Inventor: Argese, Maria, I-20122 Milano (IT); Bosone, Enrico, I-20122 Milano (IT); Clavenna, Gaetano, I-20122 Milano (IT); Giani, Roberto, I-20122 Milano (IT)
(74) Representative: Bianchetti, Giuseppe

(56) References cited:
- BE-A- 887 922
- FR-A- 2 147 812
- GB-A- 1 550 845
- Comprehensive Medicinal Chemistry, C. Hansch Ed., vol. 1, Pergamon Press, page 547
- Derwent, WPI abstract of EP-A-147171

## Description

The present invention relates to the S-carboxymethylcysteine lysine salt monohydrate and a process for the preparation thereof.

S-Carboxymethylcysteine lysine salt (SCMC-lys), of formula (Ia) 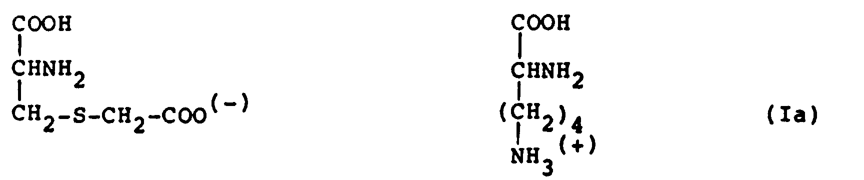
is a compound having mucolytic, bronchosecretolytic and antibronchospastic activities, which is widely used in the treatment of disorders of the respiratory tract.

Italian patent n. 1.130.972 discloses, in the general disclosure, the possibility to prepare SCMC-lys by reacting S-carboxymethylcysteine (SCMC) (racemic, dextro or levo forms) with lysine (also racemic, dextro or levo forms). However, actually said patent only discloses the preparation of SCMC-lys starting from (-)-SCMC and (±)-lysine. The salt obtained according to this process is in the anhydrous form and it is recovered by evaporation of the used hydroalcoholic solvent to dryness, which is a drawback from the point of view of working costs.

FR-A-2,147,812 discloses the preparation of the salt of (-)-SCMC with L(S)-lysine consisting in the evaporation of an aqueous solution to give the salt in hydrate, amorphous form.

Now it has been found that, using L(R)-SCMC, of formula (II) 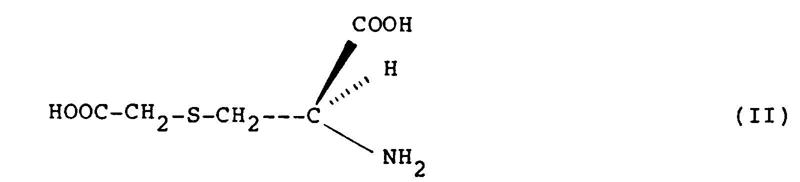
and L(S)-lysine, of formula (III) 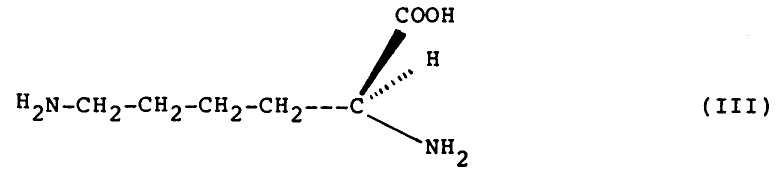
as the starting materials, under particular salification conditions, a monohydrate crystalline product (SCMC-lys monohydrate), of formula (I) 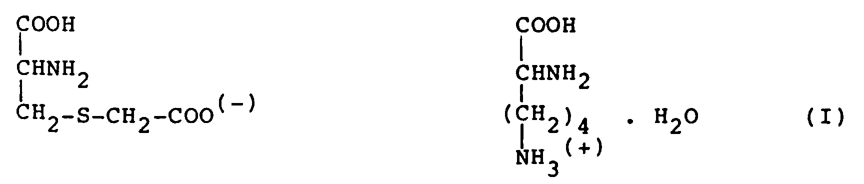
is obtained, having chemico-physical characteristics which are particularly advantageous from the technological and pharmaceutical point of view, in terms of:
a) easiness of filtration;
b) pharmaceutical ease of handling of the powder for the preparation of oral solid forms, such as granulates, capsules, tablets;
c) stability of the product;
d) higher purity.

The process of the invention consists of the following steps:
1) dissolution of L(R)-SCMC in a L(S)-lysine aqueous solution, under stirring, in almost stoichiometric ratios;
2) decolourization and filtration of the resulting solution;
3) dilution with ethanol in the amount of 2-10 volumes, preferably 4-6 volumes, with respect to the volume of the treated solution;
4) crystallization by stirring at a temperature from 15 to 30°C for 10 - 48 hours, preferably at 20-25°C for 24 - 30 hours.

The following Example further illustrates the invention.

### EXAMPLE

179.2 g (1 mole) of L(R)-S-carboxymethylcysteine are dissolved in a 50% L-lysine aqueous solution (292.4 ml, containing 1 mole of lysine) at room temperature, under stirring.

The solution is decolourized by treatment with 0.3 g of active charcoal for 30 minutes, then it is filtered and diluted with 5 volumes of ethanol. After stirring at room temperature overnight, the desired crystalline product is obtained in a 90% yield on the theoretical.
K.F. 5.2% (range from 4,0 to 6,0%).
pH (9% in H₂O) 6.0ö7.0.
[α]_{D} 1% in 2N HCl = + 9.2° (range 8.0ö10.0).

| Elementary analysis for C₁₁H₂₃N₃O₆S . H₂O | | |
|---|---|---|
| Element | Calculated % | Found % |
| C | 38.47 | 38.46 |
| H | 7.33 | 7.45 |
| N | 12.23 | 12.09 |
| S | 9.31 | 9.10 |

### DSC analysis

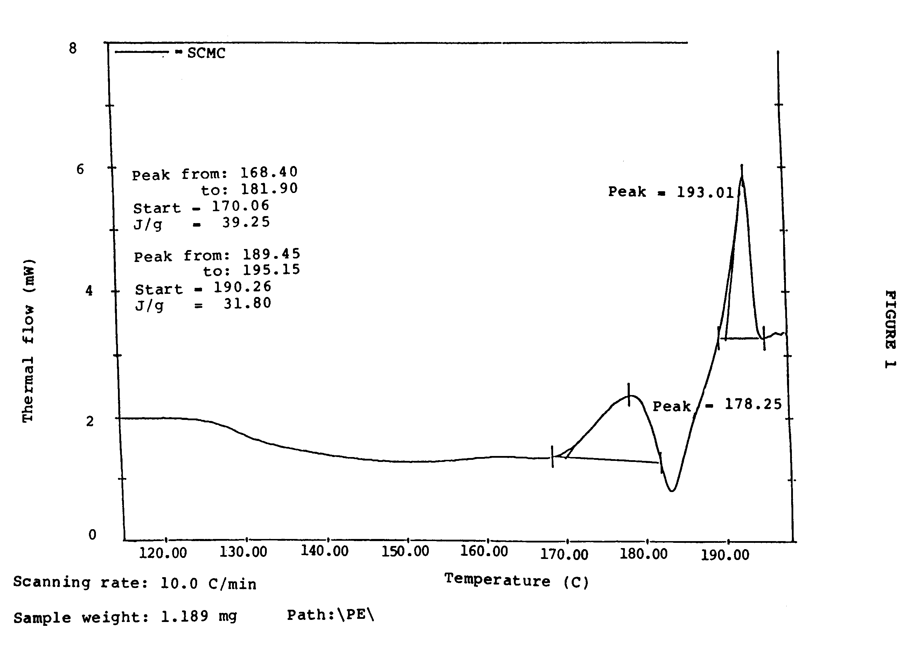

The analysis, carried out with a Perkin-Elmer DSC 7 apparatus, (Figure 1) evidences:
- an endothermic with peak at 178.25 °C
- an endothermic with peak at 193.01°C corresponding to melting.

### D.R.X. analysis

The sample was analyzed with a PW 1700 diffractometer, using the α1 and α2 copper radiations (λ = 1.54051 and λ = 1.54430 Å).

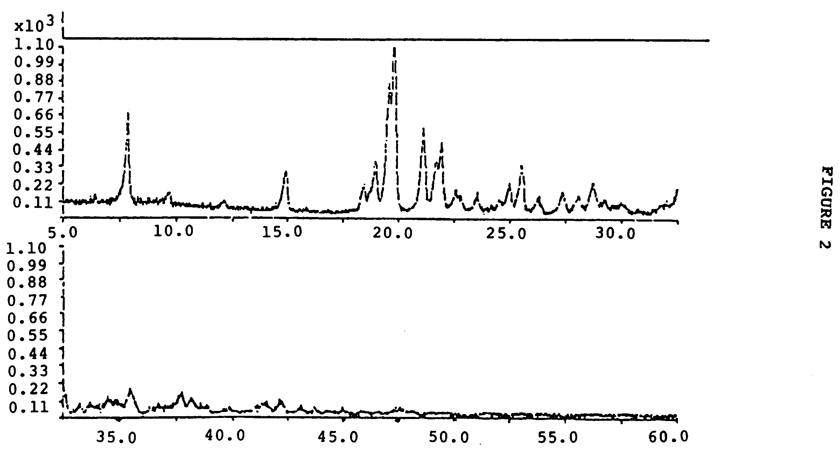

The diffractometric characteristics are reported in the spectrum tables (Figure 2).

The present invention also relates to pharmaceutical compositions, which can be administered by the oral or parenteral routes, having mucolytic, bronchosecretolytic and antibronchospastic activities, containing as the active ingredient SCMC-lys monohydrate.

Said compositions can be, for example, tablets, sugar-coated pills or capsules; effervescent powders or granules; syrups; solutions for injection; suppositories or aerosol.

## Claims

1. Crystalline S-carboxymethylcysteine lysine salt monohydrate, of formula (I) 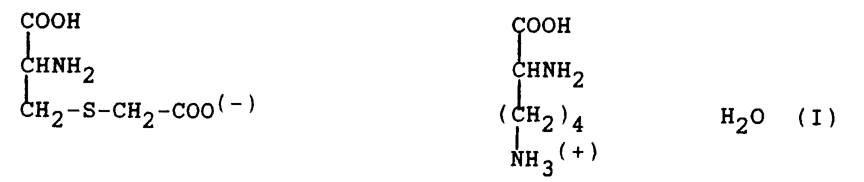 having the diffractometric characteristics reported in the following table:

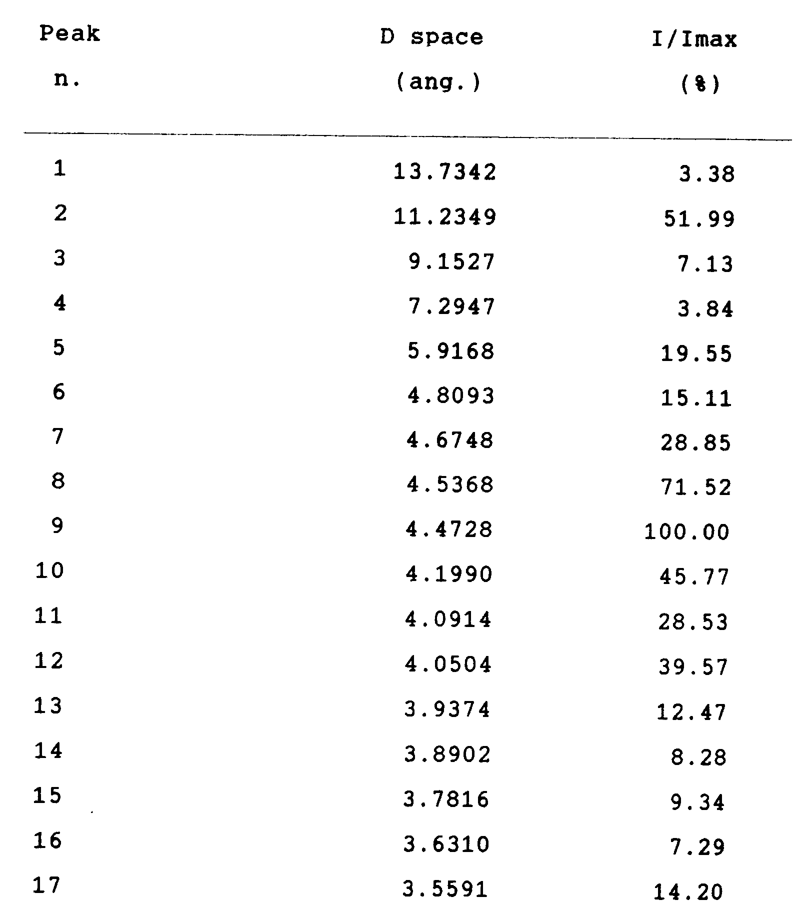

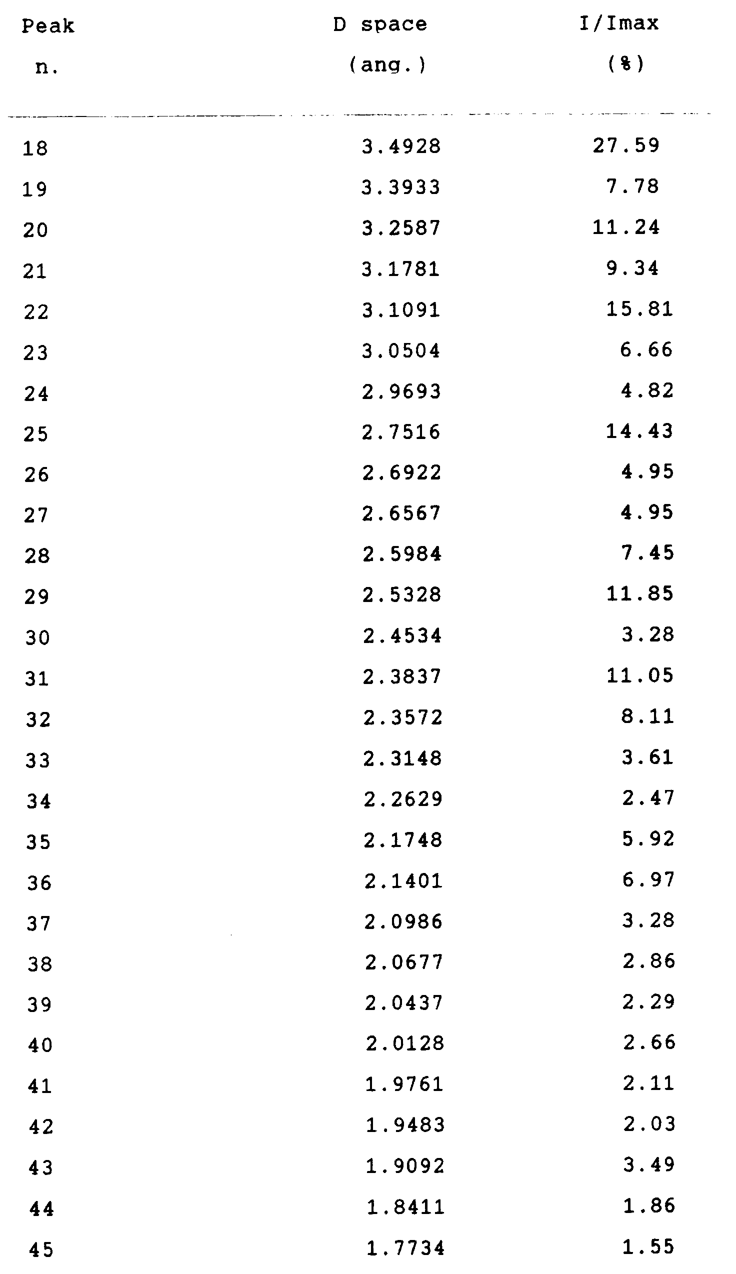

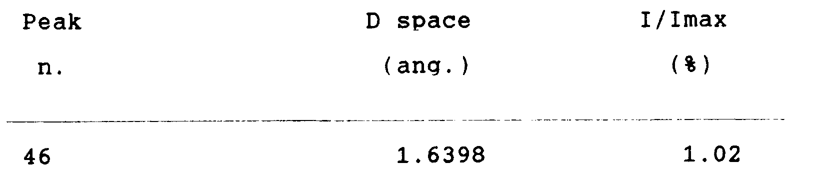

2. A process for the preparation of S-carboxymethylcysteine lysine salt monohydrate of claim 1, characterized in that L(R)-S-carboxymethylcysteine, of formula (II) 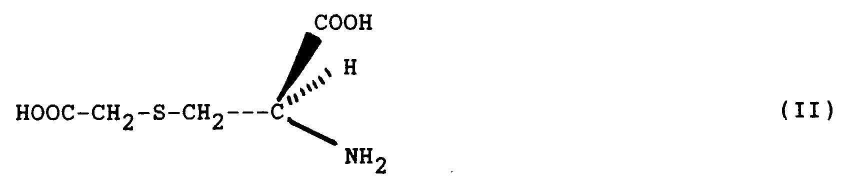 is reacted with an aqueous solution of L(S)-lysine, of formula (III) 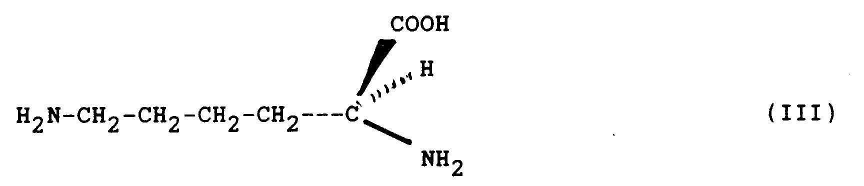 and the resulting solution is diluted with ethanol and crystallized by stirring.

3. A process according to claim 2, characterized in that the two reagents are used in stoichiometric ratios.

4. A process according to claims 2-3, characterized in that from 2 to 10 volumes of ethanol, preferably from 4 to 6 volumes, are used with respect to the volume of the treated solution.

5. A process according to claims 2-4, characterized in that the crystallization is carried out at a temperature from 15 to 30°C, preferably from 20 to 25°C.

6. A process according to claims 2-5, characterized in that the crystallization is carried out for a time from 10 to 48 hours, preferably from 24 to 30 hours.

7. Pharmaceutical compositions having mucolytic, bronchosecretolytic and antibronchospastic activities, containing as the active ingredient the compound of formula (I).

8. Pharmaceutical compositions according to claim 7, for the oral administration.

9. Pharmaceutical compositions according to claim 7, for the parenteral administration.

10. Pharmaceutical compositions according to claim 7, for the aerosol administration.

11. Pharmaceutical compositions according to claim 7, for the rectal administration.

12. S-carboxymethylcysteine lysine salt monohydrate of claim 1 as a therapeutic agent.

13. The use of S-carboxymethylcysteine lysine salt monohydrate of claim 1 for the preparation of a medicament having mucolytic, bronchosecretolytic and antibronchospastic activities.

## Patentansprüche

1. Kristallines S-Carboxymethylcystein-lysinsalz-monohydrat der Formel (I) 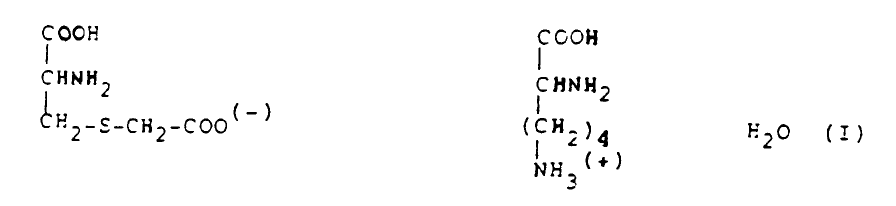 das die in der folgenden Tabelle angegebenen Beugungsmerkmale aufweist:

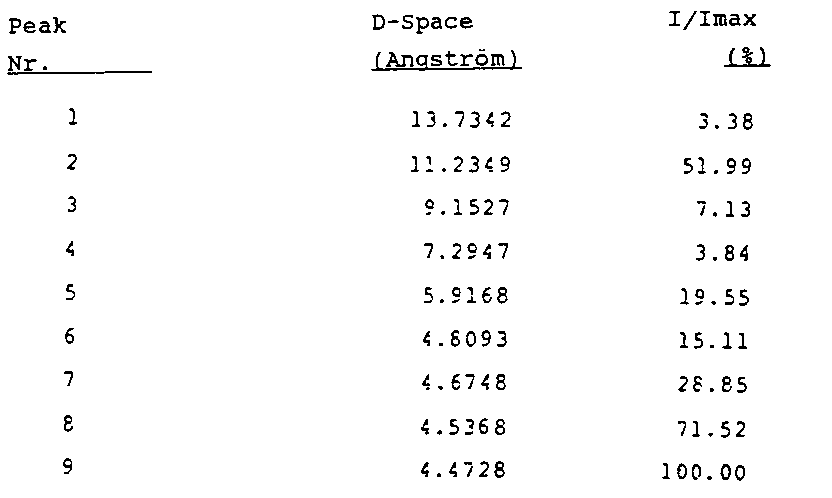

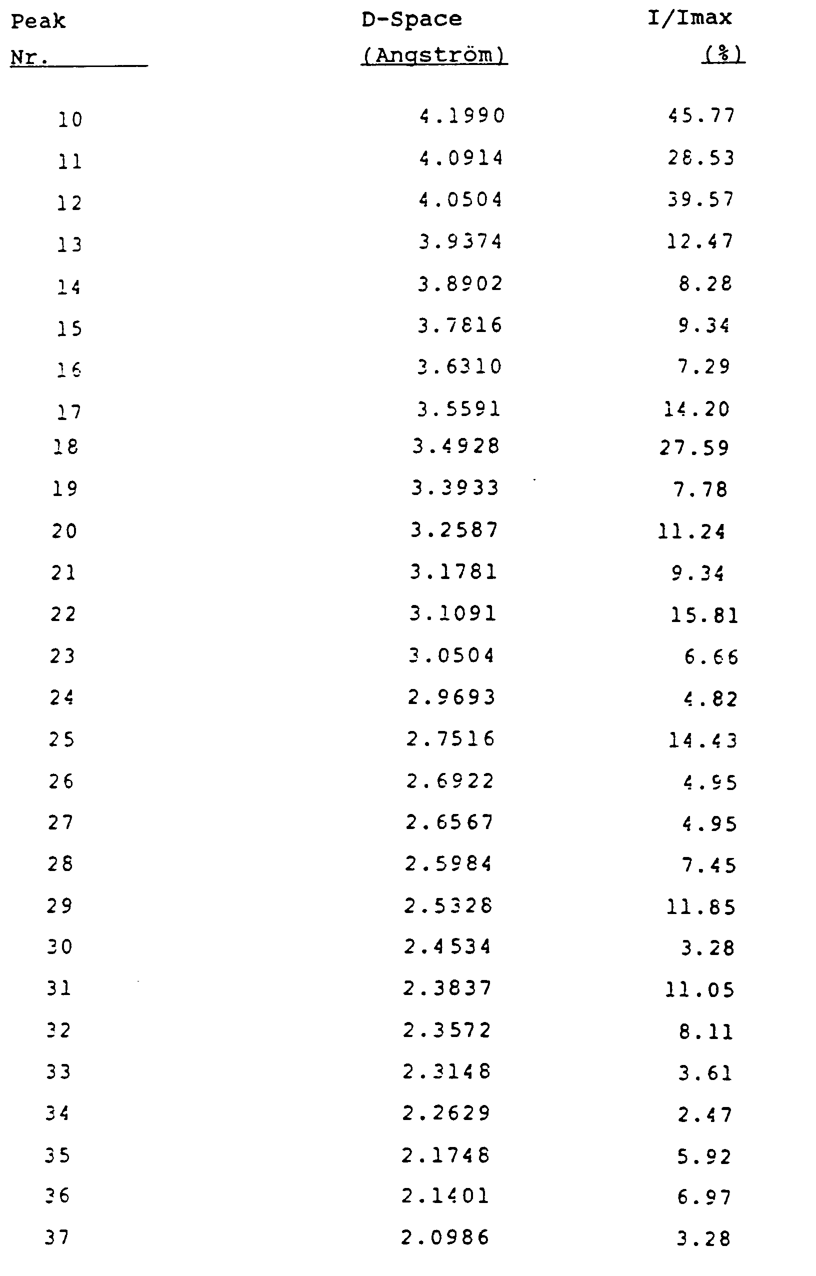

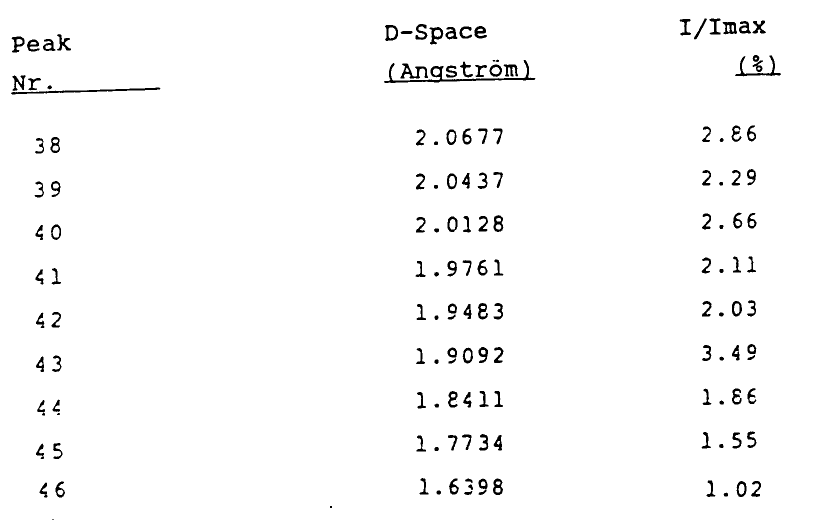

2. Verfahren zur Herstellung von S-Carboxymethylcysteinlysinsalz-monohydrat vom Anspruch 1, dadurch gekennzeichnet, daß L(R)-S-Carboxymethylcystein der Formel (II) 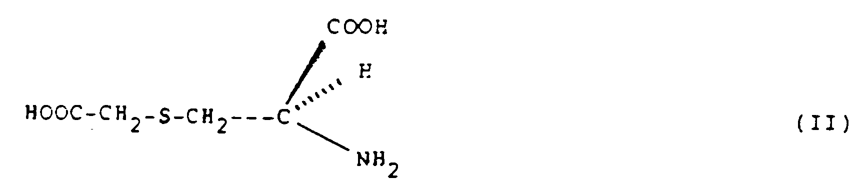 mit einer wäßrigen Lösung von L(S)-Lysin der Formel (III) umgesetzt 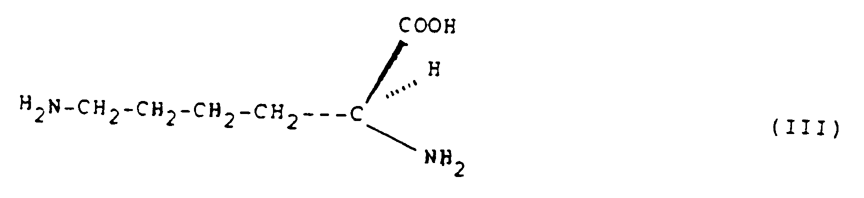 und die resultierende Lösung mit Ethanol verdünnt und durch Rühren kristallisiert wird.

3. Verfahren nach Anspruch 2, dadurch gekennzeichnet, daß die beiden Reagentien in stöchiometrischen Verhältnissen verwendet werden.

4. Verfahren nach den Ansprüchen 2 bis 3, dadurch gekennzeichnet, daß 2 bis 10, vorzugsweise 4 bis 6 Volumenteile Ethanol, bezogen auf das Volumen der behandelten Lösung, verwendet werden.

5. Verfahren nach den Ansprüchen 2 bis 4, dadurch gekennzeichnet, daß die Krisallisation bei einer Temperatur von 15 bis 30°C, vorzugsweise von 20 bis 25°C, durchgeführt wird.

6. Verfahren nach den Ansprüchen 2 bis 5, dadurch gekennzeichnet, daß die Kristallisation für eine Zeitspanne von 10 bis 48 h, vorzugsweise 24 bis 30 h, durchgeführt wird.

7. Pharmazeutische Zusammensetzungen mit mucolytischen, bronchosekretolytischen und antibronchospastischen Wirkungen (Aktivitäten), die als aktiven Bestandteil (Wirkstoff) die Verbindung der Formel (I) enthalten.

8. Pharmazeutische Zusammensetzungen nach Anspruch 7 für die orale Verabreichung.

9. Pharmazeutische Zusammensetzungen nach Anspruch 7 für die parenterale Verabreichung.

10. Pharmazeutische Zusammensetzungen nach Anspruch 7 für die Verabreichung als Aerosol.

11. Pharmazeutische Zusammensetzungen nach Anspruch 7 für die rektale Verabreichung.

12. S-Carboxymethylcystein-lysinsalz-monohydrat vom Anspruch 1 als therapeutisches Agens.

13. Verwendung von S-Carboxymethylcystein-lysinsalz-monohrdrat vom Anspruch 1 zur Herstellung eines Arzneimittels mit mucolytischen, bronchosekretolytischen und antibronchospastischen Wirkungen (Aktivitäten).

## Revendications

1. Sel de S-carboxyméthylcystéine et de lysine monohydrate, de formule (I) 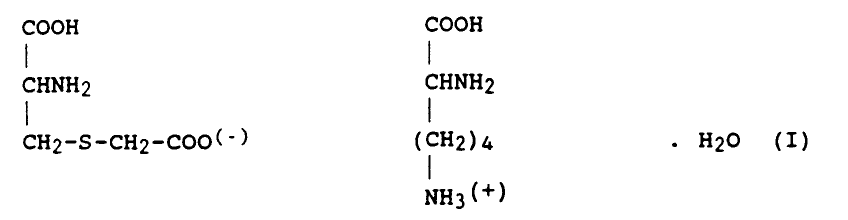 ayant les caractéristiques diffractrométriques reportées dans le tableau suivant :

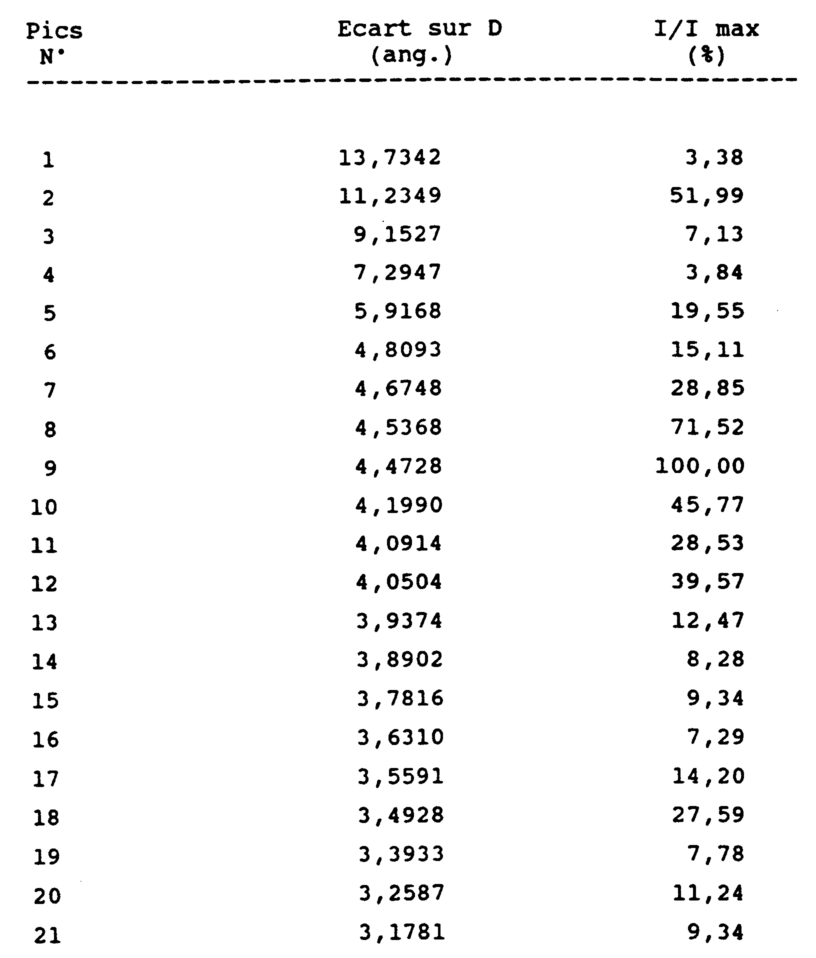

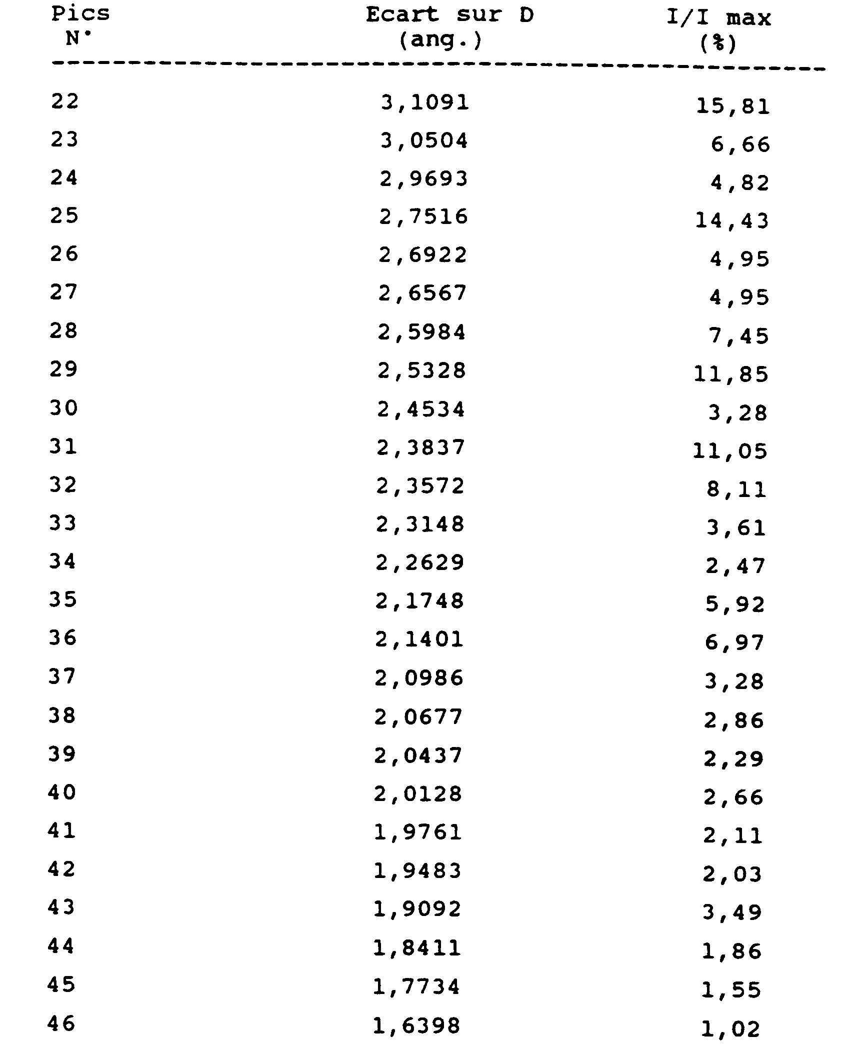

2. Procédé pour la préparation du sel de S-carboxyméthylcystéine et de lysine monohydrate de la revendication 1, caractérisé en ce que l'on fait réagir la L(R)-S-carboxyméthylcystéine, de formule (II) 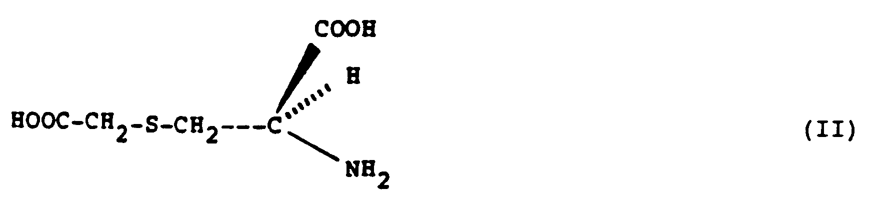 avec une solution aqueuse de L(S)-lysine, de formule (III) 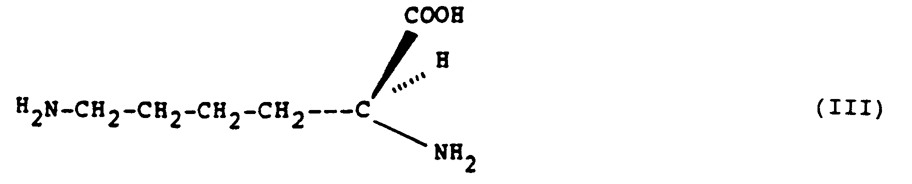 et en ce que l'on dilue la solution résultante avec de l'éthanol et on la cristallise sous agitation.

3. Procédé selon la revendication 2, caractérisé en ce que les deux réactifs sont utilisés dans des proportions stoechiométriques.

4. Procédé selon les revendications 2 à 3, caractérisé en ce qu'on utilise de 2 à 10 volumes d'éthanol, de préférence de 4 à 6 volumes, par rapport au volume de solution traitée.

5. Procédé selon les revendications 2 à 4, caractérisé en ce que la cristallisation est effectuée à une température de 15 à 30°C, de préférence de 20 à 25°C.

6. Procédé selon les revendications 2 à 5, caractérisé en ce que la cristallisation est effectuée pendant une durée de 10 à 48 heures, de préférence de 24 à 30 heures.

7. Compositions pharmaceutiques ayant des activités mucolytique, bronchosécrétolytique et antibronchospastique, contenant comme principe actif le composé de formule (I).

8. Compositions pharmaceutiques selon la revendication 7, pour administration par voie orale.

9. Compositions pharmaceutiques selon la revendication 7, pour administration par voie parentérale.

10. Compositions pharmaceutiques selon la revendication 7, pour administration par aérosol.

11. Compositions pharmaceutiques selon la revendication 7, pour administration par voie rectale.

12. Sel de S-carboxyméthylcystéine et de lysine monohydrate de la revendication 1, en tant qu'agent thérapeutique.

13. Utilisation du sel de S-carboxyméthylcystéine et de lysine monohydrate de la revendication 1, pour la préparation d'un médicament ayant des activités mucolytique, bronchosécrétolytique et antibronchospastique.
